# EUROPÄISCHE PATENTSCHRIFT

(11) **EP 3 801 401 B1**
(45) Veröffentlichungstag und Bekanntmachung des Hinweises auf die Patenterteilung: **29.06.2022**
(21) Anmeldenummer: 19726317.1
(22) Anmeldetag: 08.05.2019
(51) Int. Cl.: A61F 2/60, A61F 2/64, A61F 2/66, A61F 2/68

(54) **OBERSCHENKELPROTHESENPASSTEIL**
UPPER LEG PROSTHESIS ADAPTER
PARTIE D'ADAPTATION DE PROTHÈSE DE CUISSE

(30) Priorität: 28.05.2018 DE 102018112724
(43) Veröffentlichungstag der Anmeldung: 14.04.2021
(73) Patentinhaber: Klopf, Johannes, 97292 Holzkirchen (DE); Klopf, Matthias, 97249 Eisingen (DE)
(72) Erfinder: Klopf, Johannes, 97292 Holzkirchen (DE); Klopf, Matthias, 97249 Eisingen (DE)
(74) Vertreter: Kalkoff & Partner Patentanwälte mbB
(86) Internationale Anmeldenummer: PCT/EP2019/061844
(87) Internationale Veröffentlichungsnummer: WO 2019/228769

(56) Entgegenhaltungen:
- EP-A2- 1 447 062
- US-A- 2 568 051
- US-A1- 2015 305 895

## Beschreibung

Die Erfindung betrifft ein Oberschenkelprothesenpassteil zur Verbindung mit einem Oberschenkelschaft, aufweisend
- ein zwischen einer Standposition und einer Schwungposition verschwenkbares Kniegelenk mit einem proximalen Kniegelenkoberteil und einem distalen Kniegelenkunterteil, die über einen ventralen Gelenkarm und einen dorsalen Gelenkarm verbunden sind, wobei
   - der ventrale Gelenkarm über eine ventrale, obere Kniegelenkachse gelenkig mit dem proximalen Kniegelenkoberteil und über eine ventrale, untere Kniegelenkachse gelenkig mit dem distalen Kniegelenkunterteil und der dorsale Gelenkarm über eine dorsale, obere Kniegelenkachse gelenkig mit dem proximalen Kniegelenkoberteil und über eine dorsale, untere Kniegelenkachse gelenkig mit dem distalen Kniegelenkunterteil verbunden ist und
- einem Fußgelenk zur verschwenkbaren Verbindung eines Fußteils mit einer mit dem Kniegelenk verbundenen Unterschenkeleinheit, wie in Anspruch 1 definiert.

Oberschenkelprothesen mit einem Oberschenkelprothesenpassteil der eingangs genannten Art und einem Oberschenkelschaft sind aus der EP 1447 062 A2 bekannt. Sie werden zur prothetischen Versorgung eines Patienten verwendet und ersetzen dabei einen nicht vorhandenen Unterschenkel einschließlich des menschlichen Kniegelenks. Oberschenkelprothesen weisen dabei neben dem Prothesenkniegelenk einen Prothesenfuß auf, welcher über eine Unterschenkeleinheit mit dem Prothesenkniegelenk verbunden ist. Das Prothesenkniegelenk ist darüber hinaus zur Befestigung der Prothese an einem Oberschenkelstumpf ausgebildet, wobei hierzu üblicherweise mit dem Oberschenkelprothesenpassteil im Bereich des Kniegelenks verbundene Oberschenkelschäfte verwendet werden, in welcher der Oberschenkelstumpf eingeführt werden kann.

Während der Nutzung der Oberschenkelprothese durch den Patienten durchläuft diese mehrere Bewegungsabschnitte einer Gangphase, die auch als Gangzyklus bezeichnet wird und eine Standphase und eine Schwungphase umfasst. Die Standphase beschreibt dabei den Bereich in dem Bodenkontakt über die Oberschenkelprothese besteht, wobei während der Schwungphase kein Bodenkontakt vorliegt. Während der Standphase, die mit dem Fersenkontakt der Oberschenkelprothese beginnt und mit der Zehenablösung endet, kann sich der Patient über die Oberschenkelprothese am Untergrund abstützen. An die Standphase schließt sich die Schwungphase an, während der die Oberschenkelprothese aus der Zehenablösung bis zum Fersenkontakt durchgeschwungen wird.

Ein Problem für Patienten mit Oberschenkelprothesen besteht insbesondere darin, dass er während der Schwungphase beim Gehen mit dem gesunden Fuß eine stärkere Spitzfußstellung einnehmen muss, um den Prothesenfuß der Oberschenkelprothese beim erneuten Schritt nach vorn mit der Oberschenkelprothese durchpendeln zu lassen. Es ist insbesondere deshalb erforderlich, mit dem gesunden Fuß eine ausgeprägtere Spitzfußstellung einzunehmen, um zu verhindern, dass die Oberschenkelprothese während der Schwungphase durch bodenseitigen Kontakt am Durchschwingen gehemmt wird. Diese Ausgleichsbewegung beim Gehen ist zwingend erforderlich, um das Durchschwingen zu ermöglichen und um einer gefühlten Beinverlängerung durch die Prothese am Anfang der Schwungphase entgegenzuwirken. Da abhängig von Kniegelenktyp eine gefühlte, für das Durchschwingen erforderliche Beinverkürzung erst ab einer Kniebeugung von 30° bis 50° eintritt, bedarf es eines hohen Energieaufwands durch den Patienten, um die nötige Bodenfreiheit zu erlangen und ein Stolpern zu verhindern. Da dieser Aufwand auf Dauer nicht vom Patienten geleistet werden kann, kommt es zu einer Ausgleichsbewegung mit einer kontralateralen Spitzfußstellung und einer Zirkumduktion der Prothesenseite. Alternativ kann die Prothese kurzer ausgeführt werden, was zu einem Hinken führt. Ein weiterer Nachteil des für das Durchpendeln notwendigen Bewegungsablaufs besteht in dessen unphysiologischen Bewegungsweise, welche eine stärkere Belastung der übrigen Körperpartien, insbesondere der Wirbelsäule zur Folge hat.

Hiervon ausgehend liegt der Erfindung die Aufgabe zugrunde, ein Oberschenkelprothesenpassteil bereitzustellen, welches ein natürliches Gangbild ermöglicht und für den Patienten energieeffizienter und sturzsicherer ist.

Die Erfindung löst die Aufgabe durch ein Oberschenkelprothesenpassteil mit den Merkmalen des Anspruchs 1. Vorteilhafte Weiterbildungen der Erfindung sind in den abhängigen Ansprüchen angegeben. Im Rahmen der nachfolgenden Erörterung der Erfindung wird davon ausgegangen, dass eine Oberschenkelprothese ein beanspruchtes Oberschenkelpassteil und einen nicht beanspruchten, mit dem Oberschenkelpassteil verbundenen Oberschenkelschaft aufweist.

Das Kniegelenk des erfindungsgemäßen Oberschenkelprothesenpassteils ist durch die Verwendung eines proximalen Kniegelenkoberteils und eines distalen Kniegelenkunterteils, und deren Verbindung über einen ventralen und einen dorsalen Gelenkarm als polyzentrisches Drehgelenk ausgebildet. Damit ergibt sich der Drehpunkt des Kniegelenks aus dem Schnittpunkt der Längsachsen der Gelenkarme, welche sich bei dem ventralen Gelenkarm durch die ventrale, obere und ventrale, untere Kniegelenkachse und bei dem dorsalen Gelenkarm durch die dorsale, obere und dorsale, untere Kniegelenkachse erstrecken. Der Vorteil eines derartigen polyzentrischen Kniegelenks besteht darin, dass konstruktiv hierdurch schon der wirksame Drehpunkt des Kniegelenks so angepasst werden kann, dass dieser während der Gangphase im Bereich zwischen dem Fersenkontakt bis kurz vor der Zehenablösung, d. h. während die Oberschenkelprothese durch den Patienten beim Gehen belastet wird und sich das Kniegelenk im Streckanschlag befindet, hinter der sich im Gebrauch der Oberschenkelprothese ergebenden Belastungslinie angeordnet ist. Erst beim tatsächlichen Erreichen der Zehenablösung, d. h. am Ende der Belastungsphase der Oberschenkelprothese und mit Beginn der Schwungphase, bei der die Oberschenkelprothese unbelastet unter dem Körper durchgependelt wird, verläuft die Belastungslinie ggf. vor dem Drehpunkt des Kniegelenks, wodurch eine Beugung des Kniegelenks bewirkt wird und sich die Durchschwungphase bis zum erneuten Fersenkontakt anschließen kann. Die Ausrichtung des Drehpunkts kann auch derart erfolgen, dass während der gesamten Standphase der Drehpunkt hinter der Belastungslinie verläuft, das Kniegelenk sich im Streckanschlag befindet und dann die Schwungphase aktiv durch den Patienten über einen gewissen Widerstand eingeleitet werden muss, der von der Lage des Drehpunkts gegenüber der Belastungslinie abhängig ist.

Die erfindungsgemäße Ausgestaltung sieht vor, dass die Unterschenkeleinheit sowohl ein ventrales als auch ein dorsales Verbindungselement aufweist, welche Schub- und Zugkräfte übertragen können. Das ventrale und das dorsale Verbindungselement sind dabei über eine ventrale bzw. dorsale Fußgelenkachse gelenkig mit dem Fußteil verbunden. An ihren dem Fußteil gegenüberliegenden Enden ist das ventrale Verbindungselement mit dem distalen Kniegelenkunterteil und das dorsale Verbindungselement über die dorsale, obere Kniegelenkachse gelenkig mit dem proximalen Kniegelenkoberteil sowie dem dorsalen Gelenkarm verbunden.

Die Verbindung über die Unterschenkeleinheit ist dabei dergestalt, dass bei einer bei der Zehenablösung erfolgenden Beugung des Kniegelenks, d. h. also am Ende der Belastungsphase und zu Beginn der Schwungphase eine Dorsalextension des Fußteils erfolgt. Hierbei erfolgt eine Verschwenkung des mit herkömmlichen Prothesenfüssen verbindbaren Fußteils um die parallel zueinander verlaufenden dorsalen und ventralen Fußgelenkachsen. Die bei der Dorsalextension stattfindende Anhebung des Zehenbereichs in der Schwungposition, d. h. zu Beginn der Durchschwungphase der Oberschenkelprothese, führt zu einer Erhöhung des Abstands des Fußteils der Oberschenkelprothese von dem Boden und erlaubt es somit, auf eine Ausprägung der Spitzfußstellung mit dem gesunden Bein zu verzichten. Darüber hinaus unterstützt die durch die Kniebeugung hervorgerufene Dorsalextension ein energiesparendes Auslösen der Schwungphase. Das erfindungsgemäße Oberschenkelprothesenpassteil ermöglicht somit einen gegenüber herkömmlichen Oberschenkelprothesen natürlicheren Gang, wobei gleichzeitig auch durch die erzeugte Dorsalextension die Gangsicherheit gesteigert wird, nachdem einem möglichen Stolpern in Folge eines zu geringen Bodenabstandes des Fußteils beim Durchschwingen wirksam vorgebeugt wird.

Wie bereits eingangs dargestellt, bestimmt der Schnittpunkt der Längsachsen von ventralem und dorsalem Gelenkarm den Drehpunkt des Kniegelenks des Oberschenkelprothesenpassteils. Nach einer Weiterbildung der Erfindung ist vorgesehen, dass eine Stelleinheit vorgesehen ist, welche zur Ausrichtung des ventralen Gelenkarms gegenüber dem dorsalen Gelenkarm in der Standposition des Kniegelenks, d. h. in dessen nicht gebeugten Position ausgebildet ist.

Im Gebrauch, d. h. während der Gangphase, befindet sich die Oberschenkelprothese vom Belastungsbeginn, startend mit dem Fersenkontakt, bis zur Zehenablösung in der Standposition, in der sich das Kniegelenk im Streckanschlag befindet und nicht gebeugt ist. In Abhängigkeit von der Lage des Drehpunkts verschiebt sich erst kurz vor der Zehenablösung die Belastungslinie hinter den durch den ventralen und dorsalen Gelenkarm definierten Drehpunkt, wodurch das Kniegelenk durch Beugung in die Schwungposition überführt wird. Erfolgt keine Verschiebung des Drehpunkts hinter die Belastungslinie vor der Zehenablösung, muss die Schwungphase gegen einen Widerstand aktiv durch den Patienten eingeleitet werden. Eine Weiterbildung der Erfindung sieht vor, dass über eine Stelleinheit der ventrale Gelenkarm gegenüber dem dorsalen Gelenkarm ausgerichtet werden kann. Die Möglichkeit einer Ausrichtung von dorsalem und ventralem Gelenkarm zueinander, d. h. einer Veränderung der Winkelstellung dieser beiden Gelenkarme zueinander erlaubt es, den Schnittpunkt der Längsachsen der beiden Gelenkarme und damit den Drehpunkt des Kniegelenks zu verschieben. Die Stelleinheit bietet somit die Möglichkeit, die Oberschenkelprothese an die patientenspezifischen Anforderungen anzupassen, um so einen natürlichen Gang zu ermöglichen. Die Ausrichtung des Drehpunkts erfolgt dabei unter Berücksichtigung der gestellten Anforderungen, bspw. einer erst kurz vor der Zehenablösung zu erfolgenden Beugung sowie einer gleichzeitig stabilen Anordnung des Kniegelenks in der Standposition zum Ende der Schwungphase und mit Beginn der Standphase, die mit dem Fersenkontakt des Fußteils nach dem Durchschwingen der Oberschenkelprothese startet. Alternativ kann der Drehpunkt durch die Stelleinheit bspw. auch so ausgerichtet werden, dass eine Beugung am Ende der Standphase aktiv hervorgerufen werden muss, wobei über die Stelleinheit der hierfür zu überwindende Widerstand einstellbar ist.

Die Stelleinheit kann grundsätzlich in beliebiger Weise ausgestaltet werden, sofern mit dieser eine Festlegung der Ausrichtung der beiden Gelenkarme in der Standposition zuverlässig erreicht werden kann. Nach einer besonders vorteilhaften Ausgestaltung der Erfindung ist jedoch vorgesehen, dass die Stelleinheit ein verstellbar an dem dorsalen Gelenkarm gelagertes, in der Standposition an einer Anschlagfläche des dorsalen Verbindungselements anliegendes Stellelement, insbesondere einer Stellschraube aufweist.

Gemäß dieser Ausgestaltung der Erfindung stützt sich der dorsale Gelenkarm in der Standposition des Kniegelenks an einer Anschlagfläche des dorsalen Verbindungselements ab und legt so die Stellung des Kniegelenks in der Standposition fest. Durch die Verwendung eines verstellbar an dem dorsalen Gelenkarm angeordneten Stellelements, insbesondere einer Stellschraube, welche im Gebrauch von dem dorsalen Gelenkarm in Richtung auf das dorsale Verbindungselement vorsteht und in der Standposition an der Anschlagfläche anliegt, besteht in einfacher Weise die Möglichkeit, den Abstand zwischen der Anschlagfläche und dem dorsalen Gelenkarm im Bereich des Stellelements, insbesondere der Stellschraube, zu verändern, wodurch die Lage des dorsalen Gelenkarms gegenüber dem ventralen Gelenkarms verändert wird.

Diese Ausgestaltung der Erfindung zeichnet sich dadurch aus, dass sie in besonders einfacher und zuverlässiger Weise eine Festlegung des Drehpunkts des Kniegelenks ermöglicht. Insbesondere die Verwendung einer Stellschraube zeichnet sich dabei dadurch aus, dass sie eine sehr exakte, stufenlose und zuverlässige Festlegung dieses Drehpunkts ermöglicht, und sich dabei zugleich besonders einfach und kostengünstig ausgestalten lässt. Besonders vorteilhaft weist dabei die Anschlagfläche im Bereich des Stellelements, insbesondere der Stellschraube ein an das Ende des Stellelements, insbesondere der Stellschraube angepasste Ausnehmung auf, wodurch ein Ausweichen der Schraube zuverlässig verhindert und die Zuverlässigkeit der Positionierung des dorsalen Gelenkarms gegenüber dem ventralen Gelenkarm in ergänzender Weise gesteigert wird. Im Falle der Verwendung einer Stellschraube ist deren Gewindeverbindung bevorzugt selbsthemmend ausgebildet, sodass die eingestellte Position zuverlässig gesichert ist.

Über eine Beugung des Kniegelenks ist es im Falle eines Beugungswinkels von bis zu 90° dem Patienten auch möglich, eine sitzende Position einzunehmen, wobei während der Schwungphase das Kniegelenk im Anschluss an die Zehenablösung in der Schwungposition angeordnet ist, in der das Kniegelenk jedoch einen geringeren Beugewinkel aufweist. Nach einer vorteilhaften Ausgestaltung der Erfindung ist jedoch eine Einstellung der maximal möglichen Beugung, d. h. eine Festlegung eines maximalen Beugewinkels vorgesehen, wobei bevorzugt das Kniegelenkoberteil und das Kniegelenkunterteil derart in Wirkverbindung befindlich sind, dass eine maximale Beugung des Kniegelenks einstellbar ist. Dies kann grundsätzlich in beliebiger Weise erfolgen, wobei bevorzugt eine mechanische Begrenzung der maximalen Beugung, bspw. durch geeignete Anschlagmittel vorgesehen ist. Nach einer besonders vorteilhaften Ausgestaltung der Erfindung ist jedoch vorgesehen, dass der dorsale Gelenkarm einen in der Schwungposition an dem proximalen Kniegelenkoberteil anliegenden Beugeanschlag aufweist.

Der dorsale Gelenkarm weist gemäß dieser Ausgestaltung der Erfindung einen Anschlagkörper auf, welcher derart ausgestaltet und an dem dorsalen Gelenkarm angeordnet ist, dass eine Verschwenkung des dorsalen Gelenkarms aus der Standposition in Richtung auf die Schwungposition begrenzt ist, wobei ein an dem dorsalen Gelenkarm angeordnetes Anschlagelement an dem proximalen Kniegelenkoberteil zur Anlage gebracht wird. Diese Ausgestaltung der Erfindung zeichnet sich durch ihre besondere Einfachheit und Kompaktheit aus und ermöglicht es, in zuverlässiger Weise die maximale Verschwenkbarkeit des dorsalen Gelenkarms in Richtung der Schwungposition zu begrenzen. Die Begrenzung bietet bei einer gleichzeitig ausreichenden Dorsalextension während der Schwungphase einen wirksamen Stolperschutz. Die derart weitergebildete Oberschenkelprothese verhindert nämlich aufgrund des Beugeanschlags ein darüberhinausgehendes Verschwenken, sodass sich der Patient, wenn dieser stolpert, über das in der Schwungposition angeordnete, dann stabile Kniegelenk, zuverlässig abstützen und einen Sturz verhindern kann. Die beim Stolpern bestehende Kniebeugung in Verbindung mit einer Dorsalextension des Fußes gewährleistet überdies beim Stolpern auch einen vollflächigen Bodenkontakt mit dem Fuß, was es dem Patienten erlaubt, das Stolpern annähernd als normalen Schritt aufzunehmen, bei dem die Belastungslinie einen normalen Verlauf aufweist und sich durch Fuß und Hüfte erstreckt. Ein Sturz in Folge des Stolperns wird hierdurch wirksam verhindert.

Grundsätzlich ist die Ausgestaltung des vorzugweise vorgesehenen Beugeanschlags frei wählbar. So kann dieser in seiner einfachsten Ausgestaltung als von dem dorsalen Gelenkarm vorstehendes Anschlagelement ausgebildet sein, welches in der Schwungposition eine darüberhinausgehende Beugung verhindernd an dem proximalen Kniegelenkoberteil anliegt. Die Anordnung eines solchen Anschlagelements kann dabei unter Berücksichtigung einer ausreichenden Dorsalextension und damit einer ausreichenden Bodenfreiheit beim Durchschwingen sowie unter Berücksichtigung einer gleichzeitig ausreichenden Abstützung im Falle eines möglichen Stolperns erfolgen. Nach einer vorteilhaften Weiterbildung der Erfindung ist jedoch vorgesehen, dass der Beugeanschlag zur Festlegung der Schwungposition einstellbar an dem proximalen Kniegelenkoberteil angeordnet ist.

Die einstellbare Ausgestaltung des Beugeanschlags ermöglicht es, unter Berücksichtigung der patientenspezifischen Gegebenheiten den maximal möglichen Beugewinkel komfortabel einzustellen. Hierdurch wird die individuelle Anpassbarkeit einer derart weitergebildeten Oberschenkelprothese in ergänzender Weise gesteigert.

Nach einer weiteren Ausgestaltung der Erfindung ist ferner vorgesehen, dass das ventrale Verbindungselement und/oder das dorsale Verbindungselement längenveränderbar ausgebildet sind. Durch eine längenveränderbare Ausgestaltung eines oder beider Verbindungselemente besteht die Möglichkeit, die Oberschenkelprothese in komfortabler Weise an die patientenspezifischen Anforderungen, insbesondere die benötigte Länge anzupassen. Eine separate Einstellbarkeit von dorsalem und ventralem Verbindungselement erlaubt es dabei in besonders einfacher Weise, auf individuelle Besonderheiten des Patienten einzugehen.

Die Ausgestaltung des ventralen Verbindungselements ebenso wie des dorsalen Verbindungselements ist dabei grundsätzlich frei wählbar. Nach einer vorteilhaften Ausgestaltung der Erfindung ist jedoch vorgesehen, dass das ventrale Verbindungselement ein gelenkig mit dem Fußteil verbundenes ventrales Fußelement und eine ventrale Unterschenkelstange aufweist. Die Unterschenkelstange ist dabei in ihrer Längsachsenrichtung verstellbar mit dem ventralen Fußelement und/oder mit dem distalen Kniegelenkunterteil verbunden. Durch eine Verstellmöglichkeit der Unterschenkelstange in ihrer Längsachsenrichtung mit dem distalen Kniegelenkunterteil und/oder dem ventralen Fußelement besteht die Möglichkeit, die Längserstreckung des ventralen Verbindungselements und damit der gesamten Oberschenkelprothese in besonders einfacher Weise anzupassen. Eine mögliche Einstellbarkeit in Längsachsenrichtung kann dabei in besonders einfacher Weise dadurch realisiert werden, dass die ventrale Unterschenkelstange endseitige Gewindeabschnitte aufweist, die eine exakte axiale Justierung und Festlegung der Unterschenkelstange ermöglichen. Eine Sicherung der eingestellten Position kann dabei beispielsweise durch geeignete Sperrelemente, wie Kontermuttern erreicht werden.

Analog zu der vorstehend beschriebenen vorteilhaften Weiterbildung des ventralen Verbindungselements ist nach einer weiteren Ausgestaltung der Erfindung vorgesehen, dass das dorsale Verbindungselement ein gelenkig mit dem Fußteil verbundenes dorsales Fußelement und eine dorsale Unterschenkelstange aufweist, wobei die Unterschenkelstange in ihrer Längsachsenrichtung verstellbar mit dem dorsalen Fußelement und/oder mit dem proximalen Kniegelenkoberteil verbunden ist.

Die vorteilhafterweise vorgesehene Längenverstellbarkeit der dorsalen Unterschenkelstange ermöglicht, insbesondere in Verbindung mit der vorteilhafterweise vorgesehenen Verstellbarkeit des ventralen Verbindungselements, eine exakte Anpassung der Oberschenkelprothese an die patientenspezifischen Bedürfnisse. Die Möglichkeit der Einstellbarkeit erlaubt es auf anatomische Gegebenheiten einzugehen und eine optimal an den Patienten angepasste Oberschenkelprothese bereitzustellen.

Zur Verbindung des Oberschenkelprothesenpassteils mit einem Oberschenkelstumpf des Patienten dient das Kniegelenkoberteil, welches mit einer Schaftaufnahme zur Verbindung mit einem Oberschenkelschaft versehen ist, in den der Oberschenkelstumpf eingeführt werden kann. Nach einer Weiterbildung der Erfindung ist vorgesehen, dass das proximale Kniegelenkoberteil einen Träger und eine zwischen einer Lauf- und einer Sitzstellung verstellbare, gelenkig mit dem Träger verbundene Schaftaufnahme aufweist.

Durch diese Ausgestaltung der Erfindung weist das proximale Kniegelenkoberteil des Oberschenkelprothesenpassteils zusätzlich zu dem polyzentrisch ausgebildeten Kniegelenk ein monozentrisches Gelenk auf, welches durch die gelenkige Verbindung des Trägers mit der Schaftaufnahme gebildet wird. Die proximal zu dem Kniegelenk angeordnete Verschwenkbarkeit der Schaftaufnahme gegenüber dem Träger erlaubt es dem Benutzer im Bedarfsfall, das Knie auch über den gemäß einer vorteilhaften Weiterbildung der Erfindung vorgesehenen Beugeanschlag hinaus zu beugen. Eine weitergehende Beugung erleichtert das Sitzen oder das Treppengehen. Die Verschwenkbarkeit des Trägers gegenüber der Schaftaufnahme kann dabei ebenfalls begrenzt werden, beispielsweise durch geeignete Anschlagmittel an dem Träger und/oder der Schaftaufnahme und/oder gedämpft bzw. abgefedert ausgestaltet sein, bspw. unter Verwendung eines Hydraulikzylinders der zwischen Träger und Schaftaufnahmeangeordnet ist.

Nach einer weiteren Ausgestaltung der Erfindung ist vorgesehen, dass im Falle der vorteilhaften Ausgestaltung des proximalen Kniegelenkoberteils mit einem Träger und einer gelenkig verbundenen Schaftaufnahme der Träger über die dorsale, obere Kniegelenkachse gelenkig mit dem dorsalen Gelenkarm und dem dorsalen Verbindungselement und über die ventrale, obere Kniegelenkachse gelenkig mit der Schaftaufnahme verbunden ist. Diese Ausgestaltung der Erfindung ermöglicht eine besonders einfache und kompakte Ausgestaltung der Oberschenkelprothese und gewährleistet auch im Falle einer vorteilhafter Weise vorgesehenen Ausgestaltung des proximalen Kniegelenkoberteils mit gelenkig miteinander verbundenem Träger und Schaftaufnahme eine exakte Anpassung der Oberschenkelprothese an die Bedürfnisse des Patienten.

Grundsätzlich ist die konstruktive Ausgestaltung der gelenkigen Verbindung der Schaftaufnahme mit dem Träger frei wählbar. Hierzu zählt auch die Ausgestaltung der gelenkigen Verbindung derart, dass diese nur im Bedarfsfall aus der Lauf- in die Sitzstellung verstellt wird und im Übrigen gewährleistet ist, dass sich die Schaftaufnahme in der Laufstellung befindet und eine ausreichende Stabilität aufweist, um dem Nutzer während der Gangphase eine zuverlässige Abstützung zu gewährleisten.

Nach einer vorteilhaften Ausgestaltung der Erfindung ist vorgesehen, dass die Schaftaufnahme in der Laufstellung entriegelbar an dem Träger arretiert ist. Eine Arretierung der Schaftaufnahme an dem Träger gewährleistet im besonderen Maße, dass während der normalen Gangphase die Oberschenkelprothese zuverlässig allein über das Kniegelenk gebeugt werden kann. Einer versehentlichen Verstellung des weitergebildeten proximalen Kniegelenkoberteils aus der Lauf- in die Sitzstellung wird durch eine Arretierung wirksam vorgebeugt. Im Bedarfsfall besteht die Möglichkeit, die Arretierung durch eine Entriegelung aufzuheben, sodass dann eine Verstellung des proximalen Kniegelenkoberteils aus der Laufin die Sitzstellung in komfortabler Weise vorgenommen werden kann.

Alternativ oder ergänzend kann die Schaftaufnahme auch durch ein ausreichend dimensioniertes Federelement, bspw. eine Gasdruckfeder oder hydraulische Feder in der Laufstellung positioniert werden, sodass dann bspw. ein Sitzen oder treppab und bergab gegen einen Beugewiderstand dieses Federelements erfolgen kann.

Nach einer weiteren Ausgestaltung ist ferner vorgesehen, dass die Schaftaufnahme an dem Träger derart in Richtung auf die Laufstellung vorgespannt ist, dass die Vorspannung in der Sitzstellung blockiert ist. Eine Vorspannung der Schaftaufnahme gegenüber dem Träger in Richtung auf die Laufstellung gewährleistet im besonderen Maße, dass sich der Träger der Schaftaufnahme im Normalbetrieb in der Laufstellung befindet. Eine solche Vorspannung kann selbstständig oder ergänzend zu der vorteilhafterweise vorgesehenen Verriegelung der Schaftaufnahme in der Laufstellung eine zuverlässige Anordnung der Schaftaufnahme in der Laufstellung gewährleisten.

Gemäß dieser Weiterbildung der Erfindung ist vorgesehen, dass die Vorspannung jedoch in der Sitzstellung, nach einer Beugung des Knies über eine Verschwenkung von Träger und Schaftaufnahme um etwa 90°, nicht rückstellend wirksam ist. Die Aufhebung der Wirksamkeit der Vorspannung im Bereich der Sitzstellung verhindert, dass die Oberschenkelprothese im sitzenden Zustand selbsttätig, aufgrund einer nicht blockierten Vorspannung, in die Laufstellung gelangt. Eine solche blockierende Vorspannung kann beispielsweise durch eine geeignete Anordnung der Befestigungspunkte eines Federelements an Schaftaufnahme und bspw. distalem Kniegelenkunterteil erreicht werden, welche dazu führt, dass sich das Federelement im Bereich der Sitzstellung in einer Übertotpunktlage befindet, sodass dann die Vorspannung sogar eine Lagesicherung der Sitzstellung bewirkt.

Eine Arretierung der Schaftaufnahme in der Laufstellung an dem Träger kann grundsätzlich in beliebiger Weise erfolgen. Nach einer weiteren Ausgestaltung der Erfindung ist jedoch vorgesehen, dass an der Schaftaufnahme ein zwischen einer Verriegelungs- und einer Entriegelungsstellung verstellbarer Riegelkörper angeordnet ist, der in der Verriegelungsstellung mit einer Rastausnehmung an dem Träger in Eingriff bringbar ist.

Diese Ausgestaltung der Verriegelung stellt eine besonders einfache und kostengünstige Lösung zur Arretierung der Schaftaufnahme in der Laufstellung dar. Der Riegelkörper kann durch eine einfache manuelle Verstellung aus der Rastausnehmung heraus bewegt werden, und ermöglicht es so dem Nutzer, die Schaftaufnahme aus der Lauf- in die Sitzstellung zu verstellen. Eine erneute Verriegelung der Schaftaufnahme in der Laufstellung erfolgt dann durch eine Rückbewegung des Riegelkörpers, wobei nach einer besonders vorteilhaften Ausgestaltung der Riegelkörper in Richtung auf die Verriegelungsstellung vorgespannt ist. Dies sichert die Laufstellung in besonderer Weise und gewährleistet bei einer Rückbewegung eine selbsttätige Verriegelung.

Ausführungsbeispiele der Erfindung werden nachstehend in Bezug auf die Zeichnungen erläutert. In den Zeichnungen zeigen:
- Fig. 1: eine perspektivische Ansicht einer ersten Ausführungsform einer Oberschenkelprothese in einer Standposition;
- Fig. 2: eine Seitenansicht der Oberschenkelprothese von Figur 1 in der Standposition;
- Fig. 3: eine Rückansicht der Oberschenkelprothese von Figur 1 in der Standposition;
- Fig. 4: eine Seitenansicht der Oberschenkelprothese von Figur 1 mit einem gebeugten, in einer Schwungposition angeordneten Kniegelenk;
- Fig. 5: eine perspektivische Ansicht einer zweiten Ausführungsform einer Oberschenkelprothese am Ende einer Standphase;
- Fig. 6: eine weitere perspektivische Ansicht der Oberschenkelprothese von Figur 5 in der Standposition des Kniegelenks;
- Fig. 7: eine Seitenansicht der Oberschenkelprothese von Figur 5 in der Standposition des Kniegelenks;
- Fig. 8: eine Seitenansicht der Oberschenkelprothese von Figur 5 in einer Schwungposition des Kniegelenks;
- Fig. 9: eine Seitenansicht der Oberschenkelprothese von Figur 5 mit einem in einer Sitzstellung angeordneten proximalen Kniegelenkoberteil;
- Fig. 10: die Oberschenkelprothese von Figur 5 an einem Patienten in einer Neutralstellung;
- Fig. 11: die Oberschenkelprothese von Figur 5 an einem Patienten zum Standphasenbeginn einer Gangphase;
- Fig. 12: die Oberschenkelprothese von Figur 5 an einem Patienten während der Standphasenmitte einer Gangphase;
- Fig. 13: die Oberschenkelprothese von Figur 5 an einem Patienten zum Standphasenende einer Gangphase;
- Fig. 14: die Oberschenkelprothese von Figur 5 an einem Patienten in der Schwungphase einer Gangphase;
- Fig. 15: die Oberschenkelprothese von Figur 5 an einem Patienten in einer Sitzstellung und
- Fig. 16: die Oberschenkelprothese von Figur 5 an einem Patienten in einer Stolperstellung.

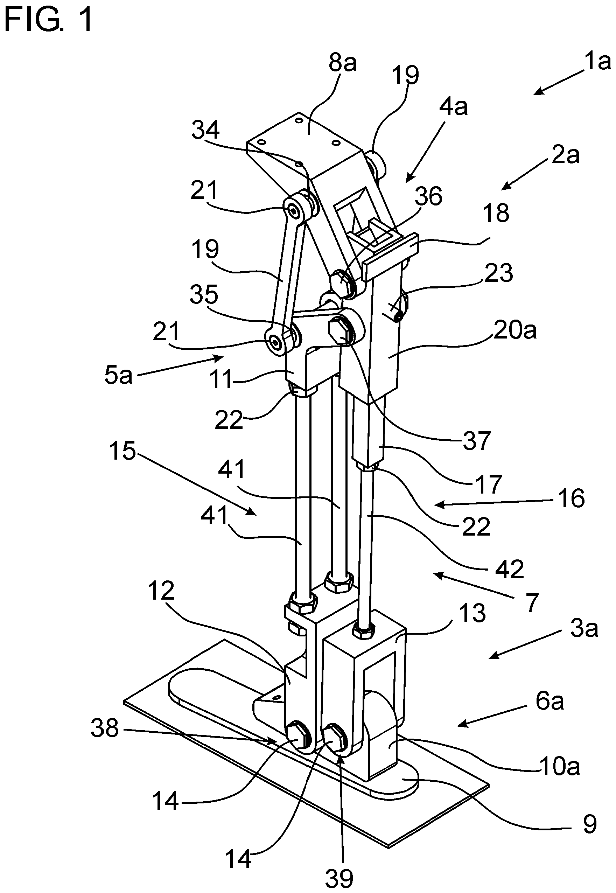

Ein in Figur 1 in einer perspektivischen Ansicht dargestelltes Oberschenkelprothesenpassteil 1a weist als wesentliche Baugruppen ein Kniegelenk 2a, eine Fußteileinheit 6a mit einem Fußgelenk 3a sowie eine Unterschenkeleinheit 7 auf.

Das Kniegelenk 2a besitzt ein proximales Kniegelenkoberteil 4a, welches gelenkig mit einem distalen Kniegelenkunterteil 5a verbunden ist. Das proximale Kniegelenkoberteil 4a weist dabei eine Schaftaufnahme 8a auf, über welches das Oberschenkelprothesenpassteil 1a über bspw. einen hier nicht dargestellten Oberschenkelschaft mit dem Patienten verbunden wird. Die Schaftaufnahme 8a ist einstückig ausgebildet und über eine ventrale, obere Kniegelenkachse 34 mit zwei an gegenüberliegenden Seiten der Schaftaufnahme 8a angeordneten ventralen Gelenkarmen 19 gelenkig verbunden. Eine Sicherung der Gelenkarme 19 an der Schaftaufnahme 8a erfolgt dabei über Senkschrauben 21. Darüber hinaus ist die einstückig ausgebildete Schaftaufnahme 8a über eine dorsale, obere Kniegelenkachse 36 gelenkig mit einem im Querschnitt U-förmigen, dorsalen Gelenkarm 20a verbunden, wobei die Position der Schaftaufnahme 8a an dem dorsalen Gelenkarm 20a über Flachkopfschrauben 14 gesichert ist.

Die Gelenkarme 19 sind an ihren der Schaftaufnahme 8a gegenüberliegenden Enden wiederum über eine durch eine Welle 24 gebildete ventrale, untere Gelenkachse 35 gelenkig mit einem Knieunterteil 11 des distalen Kniegelenkunterteils 5a verbunden, wobei auch die der Schaftaufnahme 8a gegenüberliegenden Enden der Gelenkarme 19 in ihrer Position über Senkschrauben 21 gesichert sind. Das Knieunterteil 11 ist darüber hinaus ebenfalls über eine dorsale, untere Kniegelenkachse 37 mit dem dorsalen Gelenkarm 20a verbunden. Das derart aufgebaute Kniegelenk 2a stellt somit ein polyzentrisches Gelenk dar, dessen Drehpunkt sich durch den Schnittpunkt der sich in Längsachsenrichtung durch die ventralen Gelenkarme 19 und den dorsalen Gelenkarm 20a verlaufenden Geraden ergibt.

Das vorstehend ausgebildete Kniegelenk 2a ist an seinem distalen Ende über ein ventrales Verbindungselement 15 sowie ein dorsales Verbindungselement 16 mit einem Fußteil 10a verbunden. Das dorsale Verbindungselement 16 weist dabei eine dorsale Unterschenkelstange 42 auf, die einenends mit einem Kopplungselement 17 verbunden ist, welches an seinem der dorsalen Unterschenkelstange 42 gegenüberliegenden Ende über die dorsale, obere Kniegelenkachse 36 mit der Schaftaufnahme 8a verbunden ist. Die dorsale Unterschenkelstange 42 ist dabei zur Verstellung in Längsachsenrichtung in das Kopplungselement 17 einschraubbar wobei die eingestellte Position gegenüber dem Kopplungselement 17 durch eine Sechskantmutter 22 gesichert wird.

An ihrem dem Kopplungselement 17 gegenüberliegenden Ende ist die dorsale Unterschenkelstange 42 in ein dorsales Fußelement 13 eingeschraubt, wobei über die Einschraublänge die wirksame Längserstreckung der dorsalen Unterschenkelstange 42 einstellbar ist. Eine Sicherung der Position der dorsalen Unterschenkelstange 42 an dem dorsalen Fußelement 13 erfolgt über eine Sechskantmutter 22. Das dorsale Fußelement 13 ist seinerseits über eine dorsale Fußgelenkachse 39 gelenkig und über Flachkopfschrauben 14 gesichert mit dem Fußteil 10a verbunden.

Das Knieunterteil 11 ist ferner über zwei sich parallel zueinander erstreckende ventrale Unterschenkelstangen 41 des ventralen Verbindungselements 15 mit einem ventralen Fußelement 12 verbunden, wobei sich über eine Einschraubbarkeit der ventralen Unterschenkelstangen 41 in das Knieunterteil 11 sowie das ventrale Fußelement 12 die wirksame Längserstreckung der ventralen Unterschenkelstangen 41 zwischen dem Knieunterteil 11 und dem ventralen Fußelement 12 festlegen lässt. Die eingestellte Position der ventralen Unterschenkelstangen 41 an dem Knieunterteil 11 und dem ventralen Fußelement 12 wird über Sechskantmuttern 22 gesichert. Wie auch das dorsale Fußelement 13 so ist auch das ventrale Fußelement 12 über eine ventrale Fußgelenkachse 38 gelenkig mit dem Fußteil 10a verbunden, wobei die Position des ventralen Fußelements 12 an dem Fußteil 10a über Flachkopfschrauben 14 gesichert ist. An dem Fußteil 10a ist starr eine Fußplatte 9 befestigt, über die der Bodenkontakt hergestellt wird.

Zur Festlegung des Drehpunkts des Kniegelenks 2a, welcher sich aus dem Schnittpunkt der Geraden ergibt, die sich durch die ventrale, obere Kniegelenkachse 34 und die ventrale, untere Kniegelenkachse 35 einerseits und die dorsale, obere Kniegelenkachse 36 und dorsale, untere Kniegelenkachse 37 andererseits erstrecken, ist an dem dorsalen Gelenkarm 20a ein als Stellschraube 23 ausgebildetes Stellelement angeordnet. Die Stellschraube 23 ist dabei innerhalb einer ein Gewinde aufweisenden Durchgangsbohrung des Gelenkarms 20a angeordnet und steht in Abhängigkeit von der Einschraubtiefe mit ihrem ventralen Ende in Richtung auf eine Anschlagfläche 33 des Kopplungselements 17 vor. In Abhängigkeit von der Einschraubtiefe erfolgt somit eine Verstellung der Längsausrichtung des Gelenkarms 20a und darüber hinaus über die Verbindung des Gelenkarms 20a mit dem Knieunterteil 11 eine Verstellung der Längsausrichtung der Gelenkarme 19, wodurch insgesamt der den Drehpunkt des Kniegelenks 2a definierende Schnittpunkt dieser Geraden verschoben werden kann.

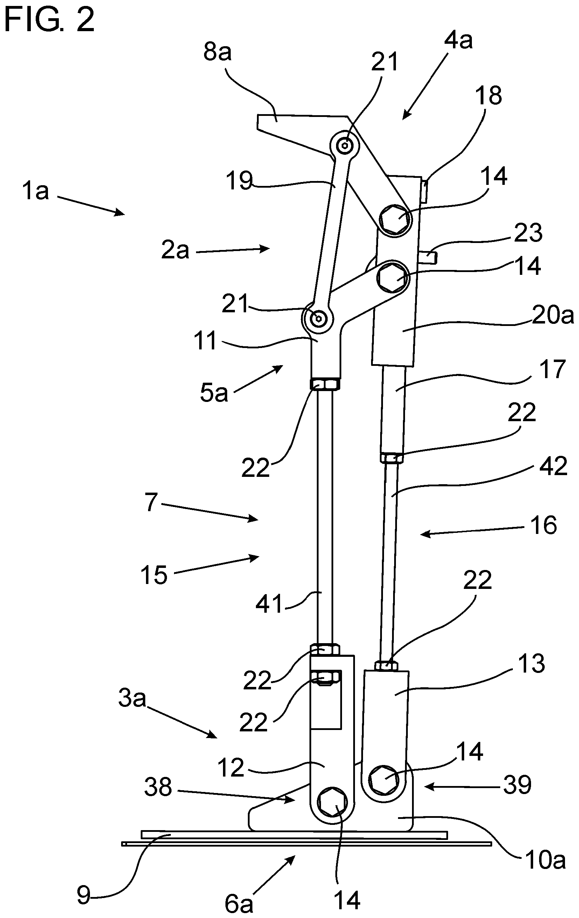

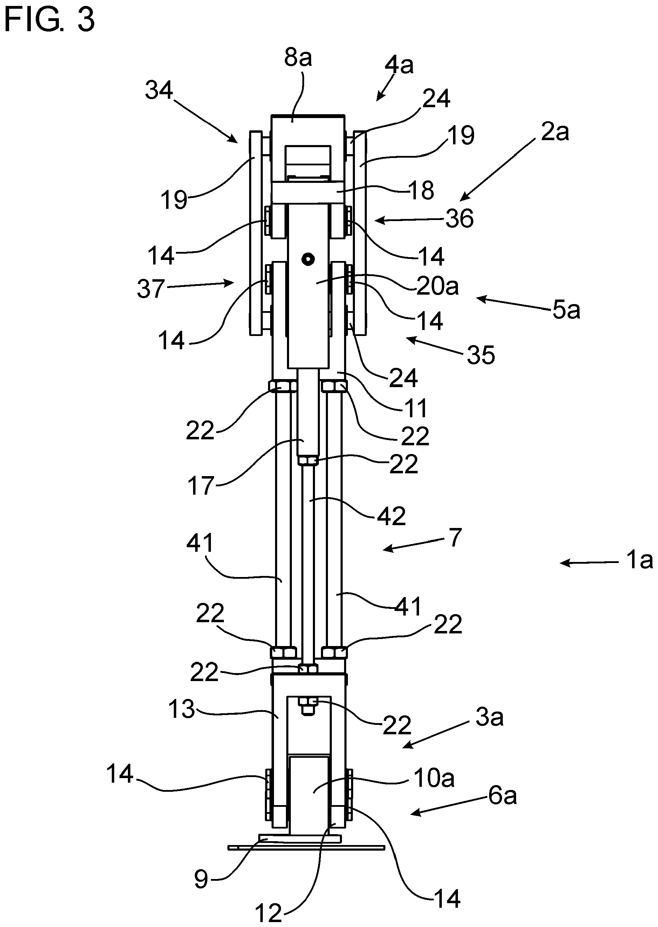

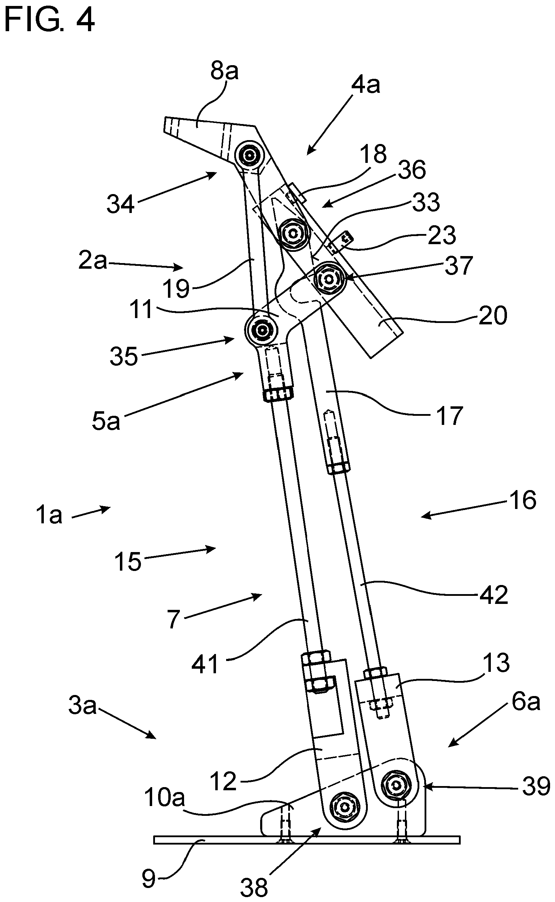

Das Oberschenkelprothesenpassteil 1a ist zwischen der in den Figuren 1 bis 3 dargestellten Standposition des Kniegelenks 2a und der in Figur 4 dargestellten Schwungposition des Kniegelenks 2a verstellbar. Im Gebrauch des Oberschenkelprothesenpassteils 1a, d. h. in seiner Position an einem Oberschenkelstumpf eines Patienten, erfolgt eine Verschwenkung des Kniegelenks 2a aus der Standposition in die Schwungposition, d. h. eine Beugung des Kniegelenks 2a, wenn sich der Vektor aus dem Körperschwerpunkt zum Kontaktpunkt am Boden des Oberschenkelprothesenpassteils 1a hinter dem Drehpunkt des Kniegelenks 2a befindet. In dieser gebeugten Position des Kniegelenks 2a befindet sich ein außenseitig an dem dorsalen Gelenkarm 20a angeordneter Beugeanschlag 18 in außenseitiger Anlage an der Schaftaufnahme 8a und beschränkt somit den maximal möglichen Beugewinkel des Kniegelenks 2a. In der in Figur 4 dargestellten gebeugten Position des Kniegelenks 2a, in der dieses sich in der Schwungposition befindet, erfolgt über die Verbindung mit dem Fußteil 10a eine Dorsalextension des Fußteils 10a, wodurch die Fußspitze angehoben wird und so ein einfaches Durchschwingen des Oberschenkelprothesenpassteils 1a möglich ist.

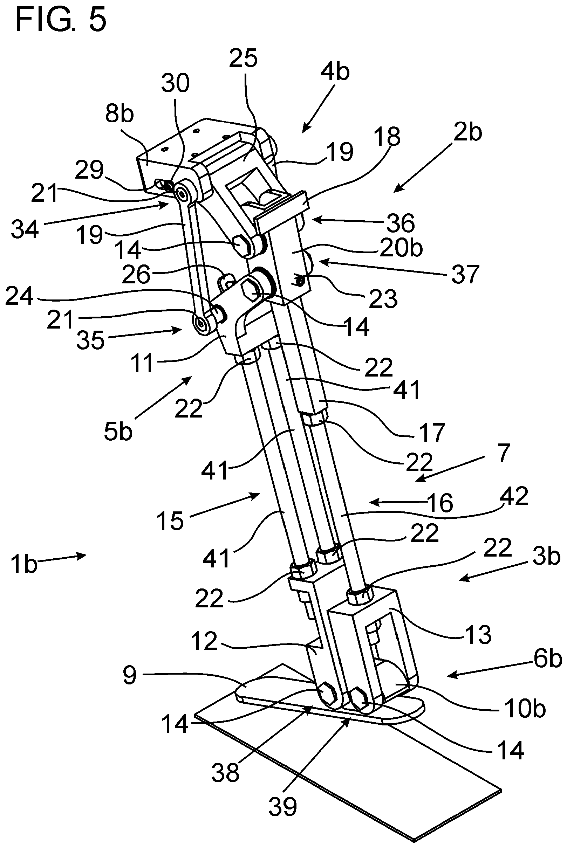

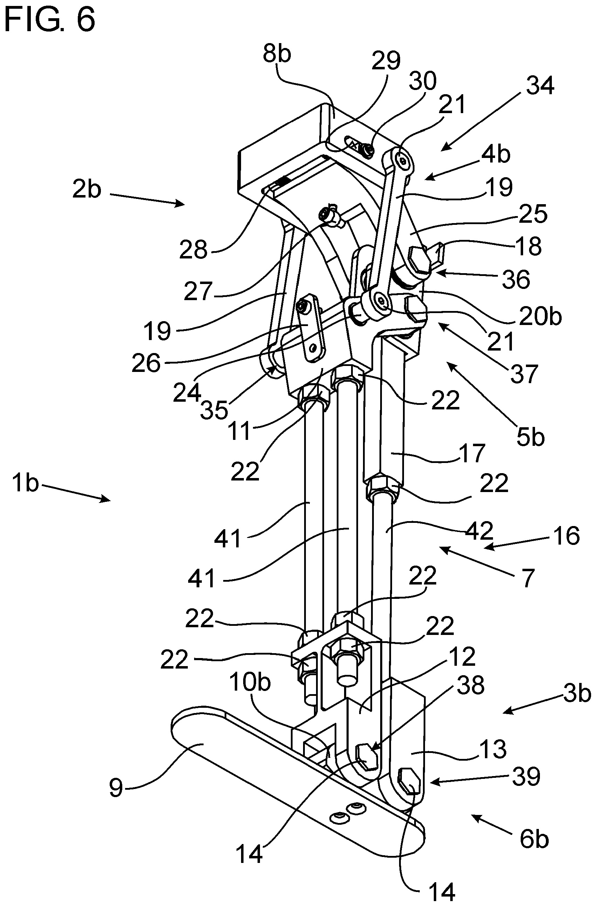

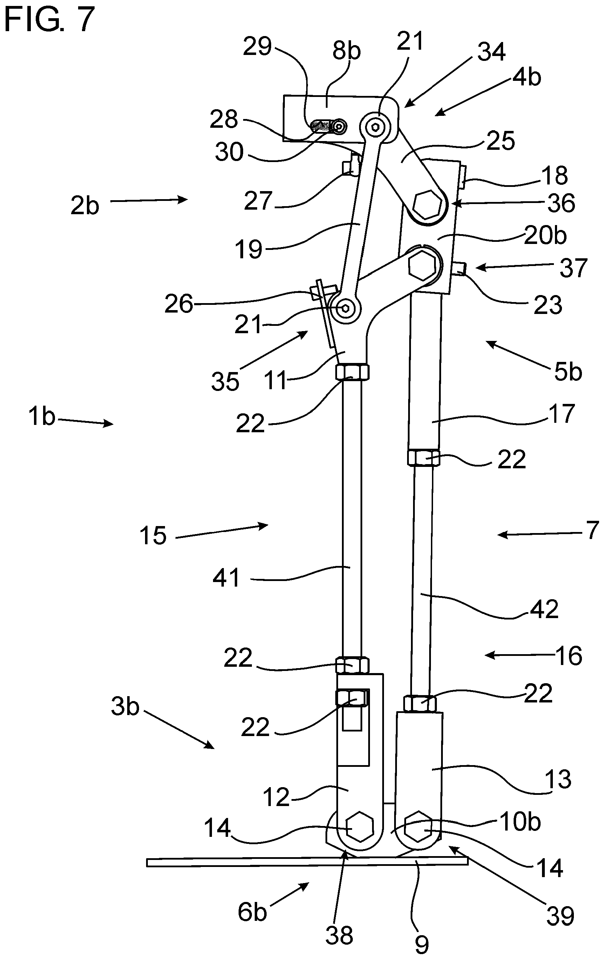

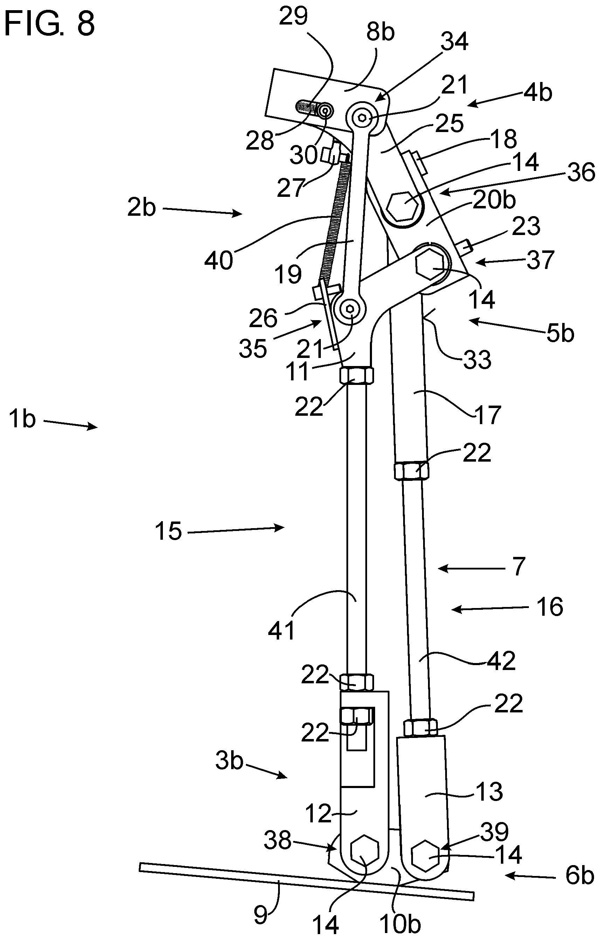

In den Figuren 5 bis 9 ist ein weiteres Ausführungsbeispiel eines Oberschenkelprothesenpassteils 1b dargestellt, das im Grundaufbau und Funktionsweise weitestgehend des in den Figuren 1 bis 4 dargestellten Oberschenkelprothesenpassteils 1a entspricht. Das Fußgelenk 3b der Fußteileinheit 6b unterscheidet sich von dem Fußgelenk 3a des Oberschenkelprothesenpassteils 1a lediglich durch eine alternative Ausgestaltung des Fußteils 10b der Fußteileinheit 6b. Im Bereich des Kniegelenks 2b weist das distale Kniegelenkunterteil 5b aufgrund einer verkürzten Ausgestaltung des Gelenkarms 20b einen geringfügigen Unterschied gegenüber dem distalen Kniegelenkunterteils 5a des Oberschenkelprothesenpassteils 1a auf. Darüber hinaus ist an dem Knieunterteil 11 eine Federaufnahme 26 angeordnet.

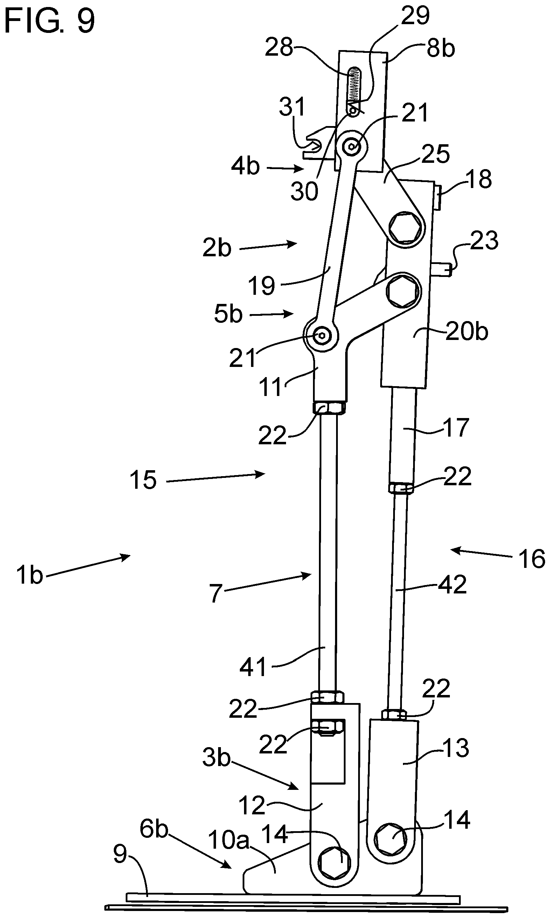

Ein wesentlicher Unterschied besteht jedoch in dem Aufbau des proximalen Kniegelenkoberteils 4b. Im Gegensatz zu dem proximalen Kniegelenkoberteil 4a der Oberschenkelprothese 1a ist die Schaftaufnahme 8b nicht einstückig ausgebildet, sondern über die ventrale, obere Kniegelenkachse 34 gelenkig mit einem Träger 25 verbunden, welcher an seinem der Schaftaufnahme 8b gegenüberliegenden Ende wiederum über die dorsale, obere Kniegelenkachse 36 gelenkig mit dem dorsalen Gelenkarm 20b und dem sich aus Kopplungselement 17 und dorsaler Unterschenkelstange 42 zusammensetzenden dorsalen Verbindungselement 16 verbunden ist. Die gelenkige Verbindung von Schaftaufnahme 8b und dorsalem Träger 25 erlaubt es, die Schaftaufnahme 8b gegenüber den Träger 25 zwischen der in den Figuren 5 bis 8 dargestellten Laufstellung und der in Figur 9 dargestellten Sitzstellung zu verschwenken.

Die Schaftaufnahme 8b ist über einen Riegelkörper 30 in der Laufstellung verriegelbar, wobei der Riegelkörper 30 durch ein Federelement 28 belastet in einer Rastausnehmung 31 angeordnet ist. Für eine Verschwenkung der Schaftaufnahme 8b um die ventrale obere Kniegelenkachse 34 ist eine Verschiebung des Riegelkörpers 30 in einem Langloch 29 aus der Rastausnehmung 31 entgegen der durch das Federelement 28 bereitgestellten Federkraft erforderlich, wobei dabei der Riegelkörper 30 in dem Langloch 29 verlagert wird. In der entriegelten Stellung ist eine Verschwenkung der Schaftaufnahme 8b aus den in den Figuren 5 - 8 dargestellten Laufstellung in die in Figur 9 dargestellte Sitzstellung möglich, bei der die Schaftaufnahme 8b um die ventrale, obere Kniegelenkachse 34 verschwenkt wird. Ein Federelement 40 erstreckt sich ferner zwischen der Federaufnahme 26 an dem Knieunterteil 11 und einer Federaufnahme 27 am Träger 25.

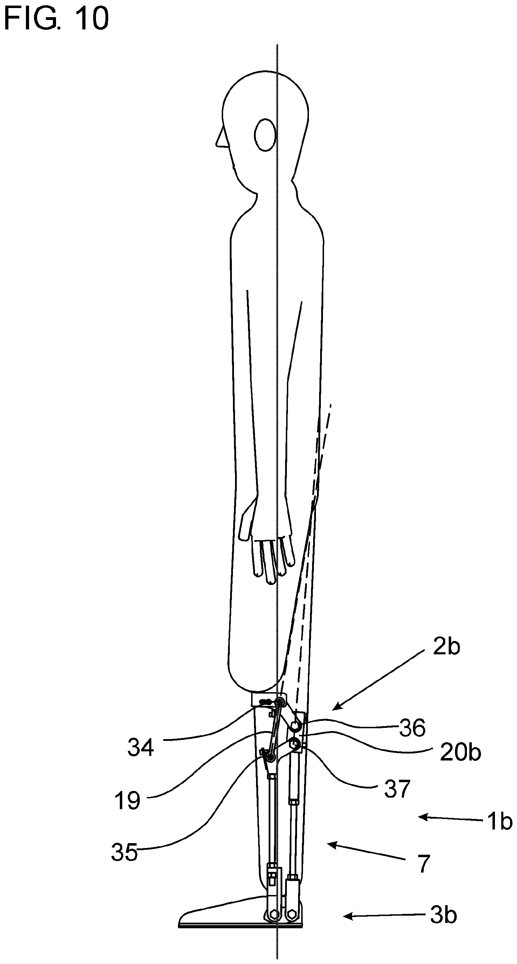

Die Funktionsweise des Oberschenkelprothesenpassteils 1b ist in den Figuren 10 bis 16 in unterschiedlichen Stellungen der Gangphase eines Patienten dargestellt. In der in Figur 10 dargestellten Neutralstellung verläuft der Vektor aus dem Körperschwerpunkt zum Kontaktpunkt am Boden vor dem Drehpunkt des Kniegelenks 2b, welcher sich aus dem Schnittpunkt der Linien (in den Figuren gestrichelt dargestellt) ergibt, die sich einerseits durch die ventrale, obere und ventrale, untere Kniegelenkachse 34, 35 und andererseits durch die dorsale obere und dorsale untere Kniegelenkachse 36, 37 erstrecken.

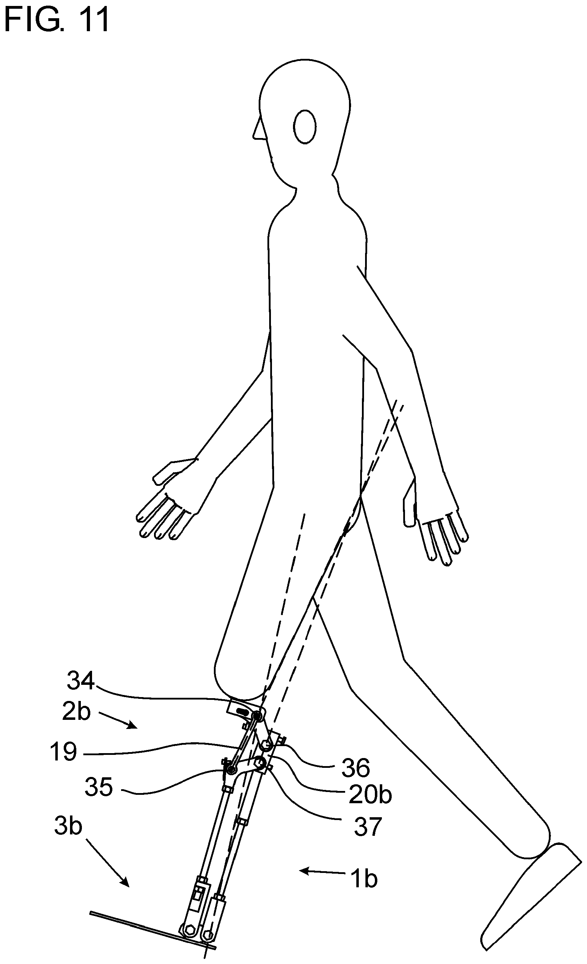

In Figur 11 ist die Position eines Patienten bei einem Fersenauftritt dargestellt. Der Vektor aus dem Körperschwerpunkt zum Kontaktpunkt am Boden befindet sich deutlich vor dem Drehpunkt und gewährleistet somit eine stabile Abstützung über das Oberschenkelprothesenpassteil 1b.

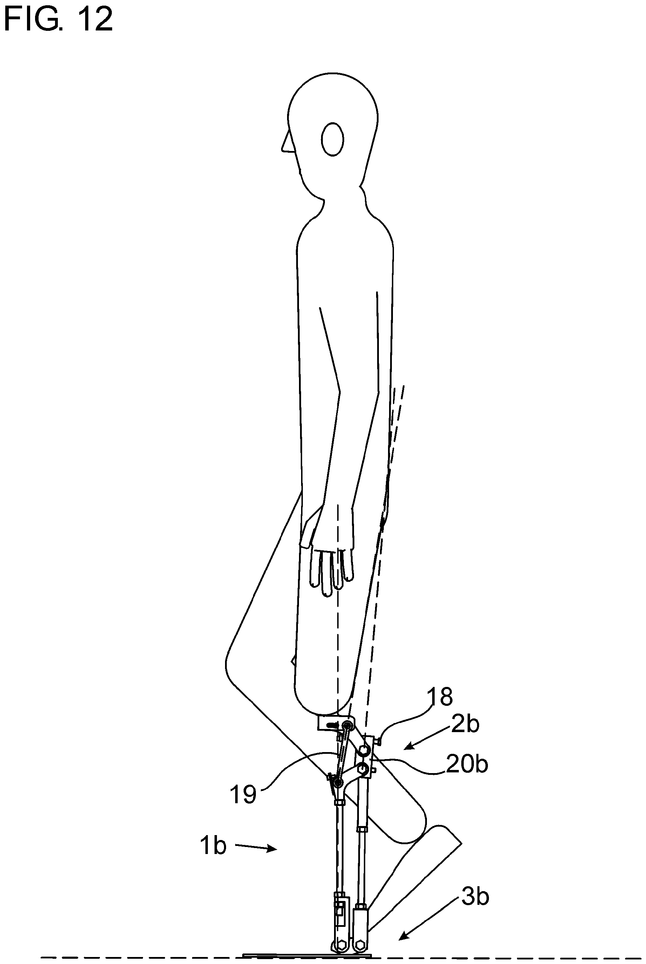

Auch in der in Figur 12 dargestellten Standphasenmitte verläuft der resultierende Vektor der Bodenreaktionskräfte deutlich vor dem Drehpunkt des Kniegelenks 2b und gewährleistet weiterhin eine stabile Abstützung über das Oberschenkelprothesenpassteil 1b.

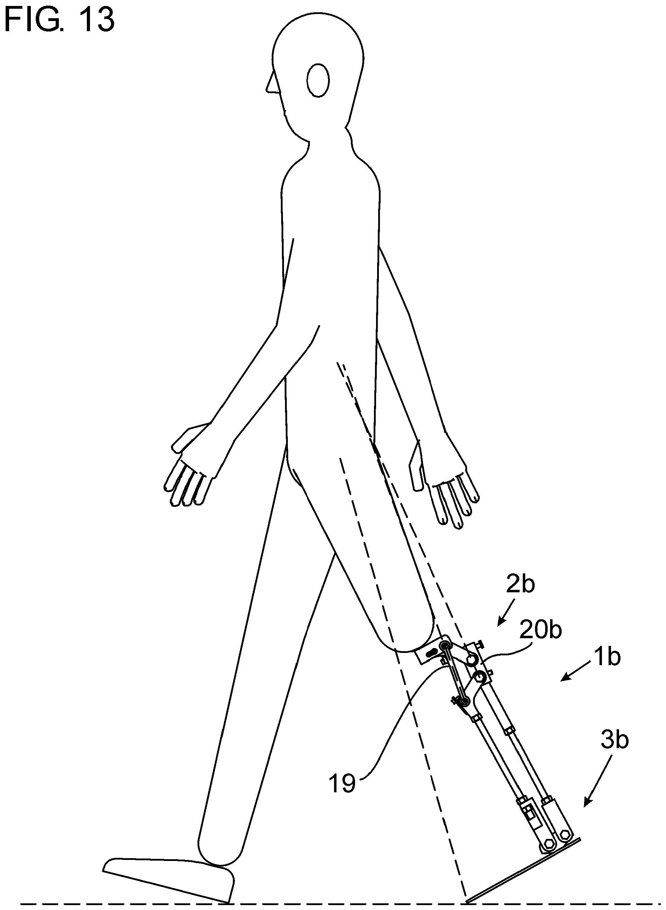

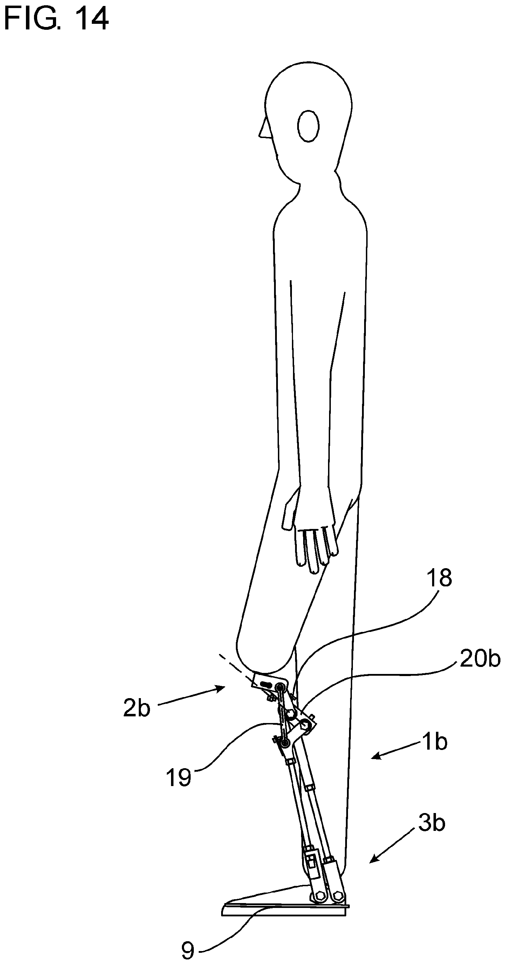

In Figur 13 ist der Zeitpunkt kurz vor der Zehenablösung dargestellt. Der momentane Drehpunkt ist auch in dieser Position immer noch in einem sicheren Bereich. Jedoch nähert sich der Drehpunkt bei Vorfußlast, verbunden mit einem geringen Hüftbeugemoment schnell dem resultierenden Vektor der Bodenreaktionskräfte und überschreitet diesen anschließend, wodurch es zu einer in Figur 14 dargestellten Beugung des Kniegelenks 2b kommt, in der das Oberschenkelprothesenpassteil 1b in der Schwungphase angeordnet ist, bei der eine Dorsalextension des Fußteils 10b aufgrund des erhöhten Bodenabstands ein sicheres Durchschwingen erlaubt.

Am Ende der Schwungphase erfolgt wiederum ein Fersenkontakt wie dieser in Figur 11 dargestellt ist, wobei dann das Kniegelenk 2b wieder in der Standposition angeordnet ist und dem Nutzer eine sichere Abstützung gewährleistet.

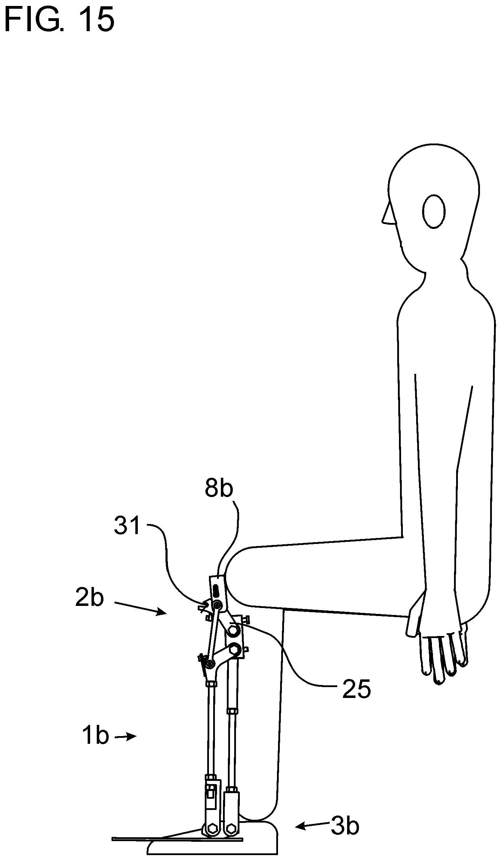

In der in Figur 15 dargestellten Position befindet sich die Schaftaufnahme 8b in einer gegenüber dem Träger 25 verschwenkten Sitzposition. Zur Erreichung dieser Position erfolgt eine Entriegelung, bei der der Riegelkörper 30 manuell aus der Rastausnehmung 31 herausbewegt wird. Nach Beendigung der Sitzposition gelangt die Schaftaufnahme 8b in seine Ausgangsposition, in der aufgrund der Federvorspannung des Riegelkörpers 30 dieser selbsttätig in die Rastausnehmung 31 gelangt.

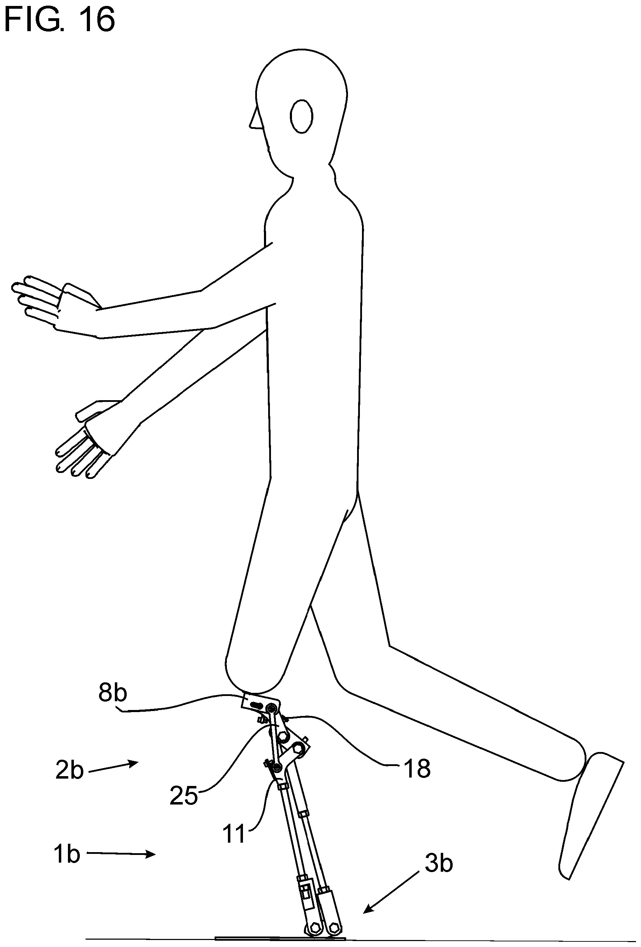

In Figur 16 ist die Situation des Oberschenkelprothesenpassteils 1b bei einem Stolpern des Patienten dargestellt. Aufgrund des Beugeanschlags 18 ist die Kniebeugung beschränkt, sodass auch bei einem Stolpern durch die durch den Beugeanschlag 18 begrenzte Beugung des Kniegelenks 2b eine stabile Abstützung in dieser Position gewährleistet ist.

**Bezugszeichenliste**

| | | | |
|---|---|---|---|
| 1a, 1b | Oberschenkelprothesenpassteil | 27 | Federaufnahme |
| 2a, 2b | Kniegelenk | 28 | Federelement |
| 3a, 3b | Fußgelenk | 29 | Langloch |
| 4a, 4b | proximales Kniegelenkoberteil | 30 | Riegelkörper |
| 5a, 5b | distales Kniegelenkunterteil | 31 | Rastausnehmung |
| 6a, 6b | Fußteileinheit | 33 | Anschlagfläche |
| 7 | Unterschenkeleinheit | 34 | ventrale, obere Kniegelenkachse |
| 8a, 8b | Schaftaufnahme | 35 | ventrale, untere Kniegelenkachse |
| 9 | Fußplatte | 36 | dorsale, obere Kniegelenkachse |
| 10a, 10b | Fußteil | 37 | dorsale, untere Kniegelenkachse |
| 11 | Knieunterteil | 38 | ventrale Fußgelenkachse |
| 12 | ventrales Fußelement | 39 | dorsale Fußgelenkachse |
| 13 | dorsales Fußelement | 40 | Federelement |
| 14 | Flachkopfschrauben | 41 | ventrale Unterschenkelstange |
| 15 | ventrales Verbindungselement | 42 | dorsale Unterschenkelstange |
| 16 | dorsales Verbindungselement | | |
| 17 | Kopplungselement | | |
| 18 | Beugeanschlag | | |
| 19 | ventraler Gelenkarm | | |
| 20a, 20b | dorsaler Gelenkarm | | |
| 21 | Senkschraube | | |
| 22 | Sechskantmutter | | |
| 23 | Stellelement/Stellschraube | | |
| 24 | Welle | | |
| 25 | Träger | | |
| 26 | Federaufnahme | | |

## Patentansprüche

1. Oberschenkelprothesenpassteil (1) zur Verbindung mit einem Oberschenkelschaft, aufweisend
- ein zwischen einer Standposition und einer Schwungposition verschwenkbares Kniegelenk (2a, 2b) mit einem proximalen Kniegelenkoberteil (4a, 4b) und einem distalen Kniegelenkunterteil (5a, 5b), die über einen ventralen Gelenkarm (19) und einen dorsalen Gelenkarm (20a, 20b) verbunden sind, wobei
- der ventrale Gelenkarm (19) über eine ventrale, obere Kniegelenkachse (34) gelenkig mit dem proximalen Kniegelenkoberteil (4a, 4b) und über eine ventrale, untere Kniegelenkachse (35) gelenkig mit dem distalen Kniegelenkunterteil (5a, 5b) und der dorsale Gelenkarm (20a, 20b) über eine dorsale, obere Kniegelenkachse (36) gelenkig mit dem proximalen Kniegelenkoberteil (4a, 4b) und über eine dorsale, untere Kniegelenkachse (37) gelenkig mit dem distalen Kniegelenkunterteil (5a, 5b) verbunden ist und
- einem Fußgelenk (3a, 3b) zur verschwenkbaren Verbindung eines Fußteils (10a, 10b) mit einer mit dem Kniegelenk (2a, 2b) verbundenen Unterschenkeleinheit (7), wobei
- die Unterschenkeleinheit (7) ein zur Übertragung von Schub- und Zugkräften ausgebildetes ventrales Verbindungselement (15) und dorsales Verbindungselement (16) aufweist, **dadurch gekennzeichnet, dass**
- das ventrale Verbindungselement (15) derart einenends über eine ventrale Fußgelenkachse (38) drehgelenkig mit dem Fußteil (10a, 10b) und anderenends mit dem distalen Kniegelenkunterteil (5a, 5b) und das dorsale Verbindungselement (16) einenends über eine dorsale Fußgelenkachse (39) mit dem Fußteil (10a, 10b) und anderenends über die dorsale, obere Kniegelenkachse (36) gelenkig mit dem proximalen Kniegelenkoberteil (4a, 4b) und dem dorsalen Gelenkarm (20a, 20b) verbunden ist, dass eine Verstellung des Kniegelenks (2a, 2b) aus der Standposition in die Schwungposition eine Dorsalextension des Fußteils (10a, 10b) bewirkt.

2. Oberschenkelprothesenpassteil nach Anspruch 1, **gekennzeichnet durch** eine in der Standposition zur Ausrichtung des ventralen Gelenkarms (19) gegenüber dem dorsalen Gelenkarm (20a, 20b) ausgebildeten Stelleinheit.

3. Oberschenkelprothesenpassteil nach Anspruch 1 oder 2, **dadurch gekennzeichnet, dass** die Stelleinheit ein verstellbar an dem dorsalen Gelenkarm (20a, 20b) gelagertes, in der Standposition an einer Anschlagfläche (33) des dorsalen Verbindungselements (16) anliegendes Stellelement, insbesondere eine Stellschraube (23) aufweist.

4. Oberschenkelprothesenpassteil nach einem oder mehreren der vorhergehenden Ansprüche, **dadurch gekennzeichnet, dass** das Kniegelenkoberteil (4a, 4b) und das Kniegelenkunterteil (5a, 5b) derart in Wirkverbindung befindlich sind, dass eine maximale Beugung des Kniegelenkes (2a, 2b) einstellbar ist.

5. Oberschenkelprothesenpassteil nach einem oder mehreren der vorhergehenden Ansprüche, **dadurch gekennzeichnet, dass** der dorsale Gelenkarm (20a, 20b) einen in der Schwungposition an dem proximalen Kniegelenkoberteil (4a, 4b) anliegenden Beugeanschlag (18) aufweist.

6. Oberschenkelprothesenpassteil nach einem oder mehreren der vorhergehenden Ansprüche, **dadurch gekennzeichnet, dass** der Beugeanschlag (18) zur Festlegung der Schwungposition einstellbar an dem proximalen Kniegelenkoberteil (4a, 4b) angeordnet ist.

7. Oberschenkelprothesenpassteil nach einem oder mehreren der vorhergehenden Ansprüche, **dadurch gekennzeichnet, dass** das ventrale Verbindungselement (15) und/oder das dorsale Verbindungselement (16) längenveränderbar ausgebildet sind.

8. Oberschenkelprothesenpassteil nach einem oder mehreren der vorhergehenden Ansprüche, **dadurch gekennzeichnet, dass** das ventrale Verbindungselement (15) ein gelenkig mit dem Fußteil (10a, 10b) verbundenes ventrales Fußelement (12) und eine ventrale Unterschenkelstange (41) aufweist, die in ihrer Längsachsenrichtung verstellbar mit dem ventralen Fußelement (12) und/oder mit dem distalen Kniegelenkunterteil (5a, 5b) verbunden ist.

9. Oberschenkelprothesenpassteil nach einem oder mehreren der vorhergehenden Ansprüche, **dadurch gekennzeichnet, dass** das dorsale Verbindungselement (15) ein gelenkig mit dem Fußteil (10a, 10b) verbundenes dorsales Fußelement (13) und eine dorsale Unterschenkelstange (42) aufweist, die in ihrer Längsachsenrichtung verstellbar mit dem dorsalen Fußelement (13) und/oder dem proximalen Kniegelenkoberteil (4a, 4b) verbunden ist.

10. Oberschenkelprothesenpassteil nach einem oder mehreren der vorhergehenden Ansprüche, **dadurch gekennzeichnet, dass** das proximale Kniegelenkoberteil (4a, 4b) einen Träger (25) und eine zwischen einer Laufstellung und einer Sitzstellung verstellbare, gelenkig mit dem Träger (25) verbundene Schaftaufnahme (8b) aufweist.

11. Oberschenkelprothesenpassteil nach einem oder mehreren der vorhergehenden Ansprüche, **dadurch gekennzeichnet, dass** der Träger (25) über die dorsale, obere Kniegelenkachse (36) gelenkig mit dem dorsalen Gelenkarm (20a, 20b) und dem dorsalen Verbindungselement (16) und über die ventrale, obere Kniegelenkachse (34) gelenkig mit der Schaftaufnahme (8b) verbunden ist.

12. Oberschenkelprothesenpassteil nach einem oder mehreren der vorhergehenden Ansprüche, **dadurch gekennzeichnet, dass** die Schaftaufnahme (8b) in der Laufstellung entriegelbar an dem Träger (25) arretiert ist.

13. Oberschenkelprothesenpassteil nach einem oder mehreren der vorhergehenden Ansprüche, **dadurch gekennzeichnet, dass** die Schaftaufnahme (8b) an dem Träger (25) derart in Richtung auf die Laufstellung vorgespannt ist, dass die Vorspannung in der Sitzstellung blockiert ist.

14. Oberschenkelprothesenpassteil nach einem oder mehreren der vorhergehenden Ansprüche, **dadurch gekennzeichnet, dass** an der Schaftaufnahme (8b) ein zwischen einer Verriegelungsstellung und einer Entriegelungsstellung verstellbarer Riegelkörper (30) angeordnet ist, der in der Verriegelungsstellung mit einer Rastausnehmung (31) an dem Träger (25) in Eingriff bringbar ist.

15. Oberschenkelprothesenpassteil nach einem oder mehreren der vorhergehenden Ansprüche, **dadurch gekennzeichnet, dass** der Riegelkörper (30) in Richtung auf die Verriegelungsstellung vorgespannt ist.

## Claims

1. An upper leg prosthesis adapter (1) for connecting to an upper leg shaft, having
- a knee joint (2a, 2b) that can be pivoted between a standing position and a swinging position with a proximal knee joint upper part (4a, 4b) and a distal knee joint lower part (5a, 5b) which are connected via a ventral joint arm (19) and a dorsal joint arm (20a, 20b), wherein
- the ventral joint arm (19) is connected in an articulated manner to the proximal knee joint upper part (4a, 4b) via a ventral, upper knee joint axis (34) and in an articulated manner via a ventral, lower knee joint axis (35) to the distal knee joint lower part (5a, 5b), and the dorsal joint arm (20a, 20b) is connected in an articulated manner to the proximal knee joint upper part (4a, 4b) via a dorsal, upper knee joint axis (36) and in an articulated manner to the distal knee joint lower part (5a, 5b) via a dorsal, lower knee joint axis (37), and
- a foot joint (3a, 3b) for pivotably connecting a foot part (10a, 10b) to a lower leg unit (7) connected to the knee joint (2a, 2b)
wherein
- the lower leg unit (7) has a ventral connection element (15) and a dorsal connection element (16) designed to transmit pushing and pulling forces, **characterized in that**
- the ventral connection element (15) is rotably hinged on one end to the foot part (10a, 10b) via a ventral foot joint axis (38) and on the other end to the distal knee joint lower part (5a, 5b), and the dorsal connection element (16) is connected on one end to the foot part (10a, 10b) via a dorsal foot joint axis (39) and on the other end in an articulated manner to the proximal knee joint upper part (4a, 4b) and the dorsal joint arm (20a, 20b) via the dorsal, upper knee joint axis (36) such that shifting the knee joint (2a, 2b) from the standing position to the swinging position causes a dorsal extension of the foot part (10a, 10b).

2. The upper leg prosthesis adapter according to claim 1, **characterized by** an adjusting unit designed to align the ventral joint arm (19) in relation to the dorsal joint arm (20a, 20b) in the standing position.

3. The upper leg prosthesis adapter according to claim 1 or 2, **characterized in that** the adjusting unit has an adjusting element, in particular an adjusting screw (23), adjustably mounted on the dorsal joint arm (20a, 20b) and abutting a stop face (33) of the dorsal connection element (16) in the standing position.

4. The upper leg prosthesis adapter according to one or more of the preceding claims, **characterized in that** the knee joint upper part (4a, 4b) and the knee joint lower part (5a, 5b) are situated in operative connection such that a maximum bending of the knee joint (2a, 2b) can be adjusted.

5. The upper leg prosthesis adapter according to one or more of the preceding claims, **characterized in that** the dorsal joint arm (20a, 20b) has a bending stop (18) abutting the proximal knee joint upper part (4a, 4b) in the swinging position.

6. The upper leg prosthesis adapter according to one or more of the preceding claims, **characterized in that** the bending stop (18) is adjustably arranged at the proximal knee joint upper part (4a, 4b) to set the swinging position.

7. The upper leg prosthesis adapter according to one or more of the preceding claims, **characterized in that** the ventral connection element (15) and/or the dorsal connection element (16) are designed to be modifiable in length.

8. The upper leg prosthesis adapter according to one or more of the preceding claims, **characterized in that** the ventral connection element (15) has a ventral foot element (12) connected in an articulated manner to the foot part (10a, 10b) and a ventral lower leg rod (41) which is adjustably connected to the ventral foot element (12) and/or to the distal knee joint lower part (5a, 5b) in its longitudinal axis direction.

9. The upper leg prosthesis adapter according to one or more of the preceding claims, **characterized in that** the dorsal connection element (16) has a dorsal foot element (13) connected in an articulated manner to the foot part (10a, 10b) and a dorsal lower leg rod (42) which is adjustably connected to the dorsal foot element (13) and/or the proximal knee joint upper part (4a, 4b) in its longitudinal axis direction.

10. The upper leg prosthesis adapter according to one or more of the preceding claims, **characterized in that** the proximal knee joint upper part (4a, 4b) has a support (25) and a shaft receiver (8b) that can be adjusted between a walking position and a sitting position and is connected in an articulated manner to the support (25).

11. The upper leg prosthesis adapter according to one or more of the preceding claims, **characterized in that** the support (25) is connected in an articulated manner to the dorsal joint arm (20a, 20b) and the dorsal connection element (16) via the dorsal, upper knee joint axis (36) and in an articulated manner to the shaft receiver (8b) via the ventral, upper knee joint axis (34).

12. The upper leg prosthesis adapter according to one or more of the preceding claims, **characterized in that** the shaft receiver (8b) is locked unlockably to the support (25) in the walking position.

13. The upper leg prosthesis adapter according to one or more of the preceding claims, **characterized in that** the shaft receiver (8b) is pretensioned on the support (25) in the direction toward the walking position such that the pretensioning is blocked in the sitting position.

14. The upper leg prosthesis adapter according to one or more of the preceding claims, **characterized in that** a locking body (30) which can be adjusted between a locked position and an unlocked position is arranged at the shaft receiver (8b), which locking body can be brought into engagement with a latching recess (31) on the support (25) in the locked position.

15. The upper leg prosthesis adapter according to one or more of the preceding claims, **characterized in that** the locking body (30) is pretensioned in the direction toward the locked position.

## Revendications

1. Partie d'adaptation de prothèse de cuisse (1) destinée à être reliée à une tige de cuisse, présentant
- une articulation de genou (2a, 2b) apte à pivoter entre une position debout et une position de pivotement, avec une partie supérieure d'articulation de genou proximale (4a, 4b) et une partie inférieure d'articulation de genou distale (5a, 5b), lesquelles sont reliées par le biais d'un bras d'articulation ventral (19) et d'un bras d'articulation dorsal (20a, 20b), dans laquelle
- le bras d'articulation ventral (19) est relié de façon articulée à la partie supérieure d'articulation de genou proximale (4a, 4b) par le biais d'un axe d'articulation de genou supérieur ventral (34) et de façon articulée à la partie inférieure d'articulation de genou distale (5a, 5b) par le biais d'un axe d'articulation de genou inférieur ventral (35) et le bras d'articulation dorsal (20a, 20b) est relié de façon articulée à la partie supérieure d'articulation de genou proximale (4a, 4b) par le biais d'un axe d'articulation de genou supérieur dorsal (36) et de façon articulée à la partie inférieure d'articulation de genou distale (5a, 5b) par le biais d'un axe d'articulation de genou inférieur dorsal (37) et
- une articulation de pied (3a, 3b) destinée à relier une partie de pied (10a, 10b) de façon pivotante à une unité de jambe (7) reliée à l'articulation de genou (2a, 2b), dans laquelle
- l'unité de jambe (7) présente un élément de liaison ventral (15) ainsi qu'un élément de liaison dorsal (16) conçus pour transmettre des forces de poussée et de traction, **caractérisée en ce que**
- l'élément de liaison ventral (15) est relié de telle façon par une extrémité à la partie de pied (10a, 10b) de façon articulée rotative par le biais d'un axe d'articulation de pied ventral (38) et par une autre extrémité à la partie inférieure d'articulation de genou distale (5a, 5b) et l'élément de liaison dorsal (16) est relié par une extrémité à la partie de pied (10a, 10b) par le biais d'un axe d'articulation de pied dorsal (39) et par une autre extrémité à la partie supérieure d'articulation de genou proximale (4a, 4b) et au bras d'articulation dorsal (20a, 20b) de façon articulée par le biais de l'axe d'articulation de genou supérieur dorsal (36), **en ce qu'**un déplacement de l'articulation de genou (2a, 2b) de la position debout vers la position de pivotement entraîne une extension dorsale de la partie de pied (10a, 10b).

2. Partie d'adaptation de prothèse de cuisse selon la revendication 1, **caractérisée par** une unité de réglage conçue pour orienter le bras d'articulation ventral (19) par rapport au bras d'articulation dorsal (20a, 20b) dans la position debout.

3. Partie d'adaptation de prothèse de cuisse selon la revendication 1 ou 2, **caractérisée en ce que** l'unité de réglage présente un élément de réglage, en particulier une vis de réglage (23), monté de façon réglable sur le bras d'articulation dorsal (20a, 20b), s'appliquant sur une surface de butée (33) de l'élément de liaison dorsal (16) dans la position debout.

4. Partie d'adaptation de prothèse de cuisse selon l'une ou plusieurs des revendications précédentes, **caractérisée en ce que** la partie supérieure d'articulation de genou (4a, 4b) et la partie inférieure d'articulation de genou (5a, 5b) sont en liaison active de telle façon qu'une flexion maximale de l'articulation de genou (2a, 2b) est réglable.

5. Partie d'adaptation de prothèse de cuisse selon l'une ou plusieurs des revendications précédentes, **caractérisée en ce que** le bras d'articulation dorsal (20a, 20b) présente une butée de flexion (18) s'appliquant sur la partie supérieure d'articulation de genou proximale (4a, 4b) dans la position de pivotement.

6. Partie d'adaptation de prothèse de cuisse selon l'une ou plusieurs des revendications précédentes, **caractérisée en ce que** la butée de flexion (18) est disposé de façon réglable sur la partie supérieure d'articulation de genou proximale (4a, 4b) pour fixer la position de pivotement.

7. Partie d'adaptation de prothèse de cuisse selon l'une ou plusieurs des revendications précédentes, **caractérisée en ce que** l'élément de liaison ventral (15) et/ou l'élément de liaison dorsal (16) sont conçus de longueur modifiable.

8. Partie d'adaptation de prothèse de cuisse selon l'une ou plusieurs des revendications précédentes, **caractérisée en ce que** l'élément de liaison ventral (15) présente un élément de pied ventral (12) relié de façon articulée à la partie de pied (10a, 10b) et une barre de jambe ventrale (41), laquelle est reliée de façon réglable à l'élément de pied ventral (12) et/ou à la partie inférieure d'articulation de genou distale (5a, 5b) dans sa direction d'axe longitudinal.

9. Partie d'adaptation de prothèse de cuisse selon l'une ou plusieurs des revendications précédentes, **caractérisée en ce que** l'élément de liaison dorsal (16) présente un élément de pied dorsal (13) relié de façon articulée à la partie de pied (10a, 10b) et une barre de jambe dorsale (42), laquelle est reliée de façon réglable à l'élément de pied dorsal (13) et/ou à la partie supérieure d'articulation de genou proximale (4a, 4b) dans sa direction d'axe longitudinal.

10. Partie d'adaptation de prothèse de cuisse selon l'une ou plusieurs des revendications précédentes, **caractérisée en ce que** la partie supérieure d'articulation de genou proximale (4a, 4b) présente un support (25) et un logement de tige (8b) relié de façon articulée au support (25) et réglable entre une position de marche et une position assise.

11. Partie d'adaptation de prothèse de cuisse selon l'une ou plusieurs des revendications précédentes, **caractérisée en ce que** le support (25) est relié de façon articulée au bras d'articulation dorsal (20a, 20b) et à l'élément de liaison dorsal (16) par le biais de l'axe d'articulation de genou supérieur dorsal (36) et de façon articulée au logement de tige (8b) par le biais de l'axe d'articulation de genou supérieur ventral (34).

12. Partie d'adaptation de prothèse de cuisse selon l'une ou plusieurs des revendications précédentes, **caractérisée en ce que** le logement de tige (8b) est arrêté de façon déverrouillable sur le support (25) dans la position de marche.

13. Partie d'adaptation de prothèse de cuisse selon l'une ou plusieurs des revendications précédentes, **caractérisée en ce que** le logement de tige (8b) est précontraint de telle façon vers la position de marche sur le support (25), que la précontrainte est bloquée dans la position assise.

14. Partie d'adaptation de prothèse de cuisse selon l'une ou plusieurs des revendications précédentes, **caractérisée en ce qu'**un corps de verrou (30) déplaçable entre une position de verrouillage et une position de déverrouillage est disposé sur le logement de tige (8b), lequel peut venir en prise avec une cavité d'encliquetage (31) sur le support (25) dans la position de verrouillage.

15. Partie d'adaptation de prothèse de cuisse selon l'une ou plusieurs des revendications précédentes, **caractérisée en ce que** le corps de verrou (30) est précontraint dans la direction vers la position de verrouillage.
